(19) Europäisches Patentamt
European Patent Office
Office européen des brevets

(11) Numéro de publication: **0 256 909 B1**

# (12) FASCICULE DE BREVET EUROPEEN

(45) Date de publication du fascicule du brevet: **31.01.90**

(51) Int. Cl.⁴: **C07C 259/06**, A61K 31/16

(21) Numéro de dépôt: **87401688.4**

(22) Date de dépôt: **20.07.87**

(54) **Acide N,N-bis-(4-fluorophényl) carbamoylacétohydroxamique et composition pharmaceutique le contenant.**

(30) Priorité: **21.07.86 FR 8610565**

(43) Date de publication de la demande:
**24.02.88 Bulletin 88/8**

(45) Mention de la délivrance du brevet:
**31.01.90 Bulletin 90/5**

(84) Etats contractants désignés:
**AT BE CH DE ES FR GB GR IT LI LU NL SE**

(56) Documents cités:
**FR-A- 2 345 430**

(73) Titulaire: **LABORATOIRE L. LAFON Société anonyme dite:, 1 rue Georges Médéric, F-94701 Maisons-Alfort(FR)**

(72) Inventeur: **Lafon, Louis, 5, rue de l'Alboni, F-75016 Paris(FR)**

(74) Mandataire: **Gillard, Marie-Louise et al, Cabinet Beau de Loménie 55, Rue d'Amsterdam, F-75008 Paris(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention a pour objet un nouvel acide acétohydroxamique et les compositions pharmaceutiques le contenant.

De nombreux acides acétohydroxamiques ont déjà été révélés dans la littérature. C'est ainsi que dans la demande de brevet français n° 77 06997 de la Demanderesse on a décrit une vaste famille de composés répondant à la formule générale :

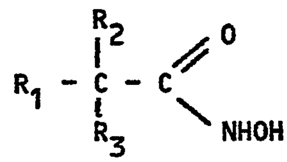

(I)

qui, dans le cas où $R_2$et $R_3$ représentent chacun un atome d'hydrogène définit un grand nombre d'acides acétohydroxamiques.

Dans cette demande antérieure, on indiquait également que certains des composés décrits présentaient une activité thérapeutique.

On a maintenant découvert que, parmi ces acides hydroxamiques, l'acide N,N-bis-(4-fluorophényl)carbamoylacétohydroxamique présente des propriétés stimulantes particulièrement intéressantes.

Dans sa grande généralité, la demande de brevet français n°77 06997 englobe le composé de l'invention, mais celui-ci n'y est cependant jamais spécifiquement décrit.

La présente invention concerne donc d'abord en tant que produit nouveau l'acide acétohydroxamique précité ; elle concerne également les compositions thérapeutiques, présentant notamment un effet antidépresseur stimulant, contenant ce nouvel acide.

Dans la demande de brevet français antérieure n° 77 06997, le composé spécifiquement décrit, le plus proche de l'invention, porte le numéro de Code CRL 40385. Ce composé est doué d'un effet anti-fatigue, mis en évidence par un ensemble de tests dont les résultats sont donnés dans ce document antérieur.

L'acide N,N-bis-(4-fluorophényl)carbamoylacétohydroxamique selon l'invention, ne se distingue du composé CRL 40385 que par la présence d'un atome de fluor en position 4, sur chacun des noyaux benzéniques.Malgré cette très grande proximité de structure, on a découvert que ce composé présentait un effet antidépresseur stimulant, qui était a priori tout à fait inattendu, puisque le CRL 40385 était doué d'un effet antifatigue mais pas d'un effet stimulant du type amphétaminique.

L'invention est donc basée sur la découverte tout à fait surprenante que ces deux composés très proches, de par leur structure chimique présentent des activités différentes et imprévisibles.

On décrira tout d'abord à titre non limitatif un procédé de préparation du nouveau composé selon l'invention.

Exemple de préparation de l'acide N,N-bis-(4-fluorophényl) carbamoylacétohydroxamique (CRL 41131).

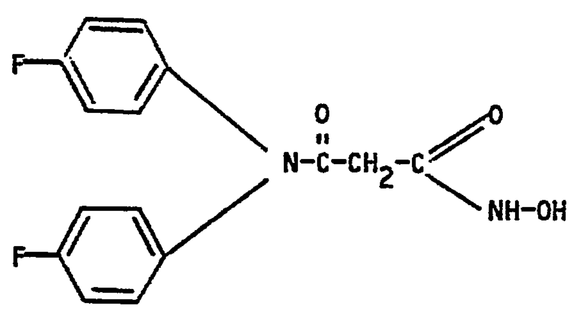

$C_{15}H_{12}O_3N_2F_2$ $P_M = 306$

1) N,N-bis-(4-fluorophényl)carbamoylacétate de méthyle.

On fait réagir à 20°C pendant 72 h 12,3 g (0,09 mole) de chlorure de méthylmalonyle sur 16,4 g (0,08 mole) de 4,4'-difluorodiphénylamine en solution dans 120 ml de benzène. On évapore à sec sous vide, reprend avec 50 ml d'éther isopropylique, essore et sèche. On obtient l'ester méthylique (F. = 84°C) avec un rendement de 95%.

1. Acetohyroxamic acid, characterized in that it the N,N-bis-(4-fluorophenyl) carbanoylacetohyroxamic acid of formula:

2. Therapeutic composition, characterized in that it contains in combination with a pharamcologically acceptalbe excipient, the N,N-bis-(4-fluorophenyl) carbamoylacetohydroxamic acid as active agent, particularly as stimulant antidepressant.

2) Acide N,N-bis-(4-fluorophényl)carbamoylacétohydroxamique

Dans une solution méthanolique d'hydroxylamine, préparée avec 3,5 g (0,05 mole) de chlorhydrate d'hydroxylamine et 2,3 g (0,1 Atg) de sodium dans 160 ml de méthanol, on ajoute 14,6 g (0,048 mole) de l'ester précédent (1); après 3 h à 20°C, on évapore le méthanol sous vide, reprend avec 200 ml d'eau, extrait deux fois à l'éther, précipite avec HCl concentré, essore, sèche et recristallise dans l'acétate d'éthyle.

On obtient le CRL 41131 avec un rendement de 72%.

C'est une poudre créme, soluble dans les alcools, l'acétone, l'acétate d'éthyle, peu soluble dans l'éther, insoluble dans l'eau, l'éther isopropylique, etc.

Il fond à 90-91°C.

Propriétés pharmacologiques du CRL 41131.

Le CRL 41131, en suspension dans une solution de gomme arabique à 5%, a été administré par voie intrapéritonéale sous un volume de 20 ml/kg chez la souris (mâle, NMRI, Evic Ceba) et de 5 ml/kg chez le rat (mâle, $CD_1$, Sprague Dawley, Charles River).

1 - Prétoxicité (3 souris par dose) - 128 et 256 mg/kg - à partir de 30 min : excitation; à 24 h, l'excitation est encore présente, et présence de stéréotypies.

- 512 mg/kg - sédation, crampes abdominales, diminution de la fréquence respiratoire pendant 30 min, puis excitation.

A 24 h : excitation et stéréotypies.

- 1024 mg/kg - sédation, crampes abdominales, diminution de la fréquence respiratoire pendant 30 min, puis excitation.

A 24 h : excitation, stéréotypies et convulsions.

Aucune mortalité n'a été constatée.

2) Comportement global et réactivités.

Des lots de 3 animaux sont observés avant, puis 15 min, 30 min, 1 h, 2 h, 3 h et 24 h après l'administration de CRL 41131.

1 - Souris - 8 et 32 mg/kg - Comportement, réactivités, variations du diamètre pupillaire et de la température comparables à ceux du lot témoin.

- 128 mg/kg - excitation (1/3) pendant 1 h,
- 512 mg/kg - excitation (1/3 pendant 1 h, 2/3 entre 2 et 3 h, et 3/3 à 24 h).
- mouvements d'apparence stéréotypée, augmentation de la réaction de peur et de la réactivité au toucher à 24 h.
- hypothermie, entre 2 h et 3 h (-1°2).

1 - Rat

- 4, 16 et 64 mg/kg - pas de modification nette du comportement et des réactivités.
- 256 mg/kg - excitation et augmentation de la réaction de peur (entre 2 h et 24 h).
- stéréotypies, augmentation de la réactivité au toucher en 24 h.

3) Recherches de mouvements stéréotypés.

Des lots de 6 rats reçoivent une injection de CRL 41131 ou d'amphétamine, puis sont immédiatement placés dans des boîtes de petites dimensions (20 × 10 × 10 cm) où leur comportement stéréotypé est coté de 0 à 3 jusqu'à extinction de l'effet.

Dès la plus faible dose utilisée (64 mg/kg), le CRL 41131 provoque l'apparition de mouvements stéréotypés chez le rat. Cet effet ne peut être qu'imparfaitement estimé au cours de la période habituelle d'observation de 7 h après administration du produit : le maximum des stéréotypies se situant probablement plus tard. En effet, après l'interruption de l'observation due à la période nocturne, les animaux présentent le lendemain matin (soit 22 h 30 min après administration du CRL 41131) des mouvements stéréotypés très intenses (cotes : 1 à 64 mg/kg, 2 à 128 mg/kg, 3 à 256 mg/kg) persistant pendant 26 h 30 min à 64 mg/kg et plus de 30 h à 128 et 256 mg/kg.

4) Interaction avec l'apomorphine.

1 - Souris

Des lots de 6 souris reçoivent le CRL 41 131 30 min avant l'injection sous-cutanée d'apomorphine, à la dose de 1 ou 16 mg/kg.

a) Apomorphine 1 mg/kg

b) Apomorphine 16 mg/kg

Le CRL 41131 ne s'oppose pas à l'action hypothermisante induite par l'apomorphine; les comportements de verticalisation et de stéréotypies ne sont pas modifiés.

2 - Rat

Le CRL 41131 est administré à des lots de 6 rats 30 min avant l'injection sous-cutanée de 0,5 mg/kg d'apomorphine.

Aux doses de 64 mais surtout 256 mg/kg, le CRL 41134 entraîne une potentialisation des stéréotypies induites par l'apomorphine.

5) Interaction avec l'amphétamine.

L'amphétamine (2 mg/kg) est injectée par voie intrapéritonéale à des lots de 6 rats, 30 min après l'administration de CRL 41131.

Aux doses de 64 et surtout 256 mg/kg, le CRL 41131 entraîne une potentialisation importante des stéréotypies amphétaminiques.

6) Interaction avec la réserpine.

Quatre heures après l'injection intrapéritonéale de 2,5 mg/kg de réserpine, des lots de 6 souris reçoivent le CRL 41131.

1-Action sur la température

2-Action sur le ptôsis

Aux doses de 128 et 512 mg/kg, le CRL 41131 s'oppose tardivement (24 h) à l'hypothermie et au ptôsis réserpiniques.

7) Interaction avec l'oxotrémorine.

Le CRL 41131 est administré à des lots de 6 souris 30 min avant l'injection intrapéritonéale de 0,5 mg/kg d'oxotrémorine.

1 - Action sur la température

Le CRL 41131 ne modifie pas l'action hypothermisante de l'oxotrémorine.

2 - Action sur les tremblements

Le CRL 41131 ne modifie pas l'intensité des tremblements provoqués par l'oxotrémorine.

3 - Action sur les symptômes cholinergiques périphériques.

Le CRL 41131 ne modifie pas les signes de stimulation cholinergique périphérique dus à l'oxotrémorine.

8) Action sur le test quatre plaques, la traction et l'électrochoc.

Le test est pratiqué sur des lots de 10 souris, 30 min après l'administration de CRL 41131.

Le CRL 41131 n'entraîne pas d'augmentation nette du nombre de passages punis; il ne provoque pas l'incapacité motrice majeure. Aux doses de 32, 128 et 512 mg/kg, le CRL 41131 aggrave modérément les effets létaux de l'électrochoc.

9) Action sur la motilité spontanée.

Après avoir reçu le CRL 41131, les souris (6 par dose, 12 témoins) sont placées en actimètre où leur motilité est enregistrée pendant 30 min.

1) CRL 41131 administré 30 min avant passage dans l'actimètre.

Aux doses de 8, 32, 128 et 512 mg/kg, le CRL 41131 augmente modérément l'activité motrice spontanée de la souris. A la plus forte dose (512 mg/kg), l'hypermotilité est moins importante qu'à 128 mg/kg.

2) CRL 41131 administré 2 h avant passage dans l'actimètre.

Dès la dose de 8 mg/kg, le CRL 41131 augmente l'activité motrice spontanée de la souris. Aux doses de 128 et 512 mg/kg, cette augmentation est plus nette.

3) CRL 41131 administré 4 h avant passage dans l'actimètre.

Aux doses de 128 et 512 mg/kg, le CRL 41131 augmente la motilité spontanée de la souris.

4) CRL 41131 administré 24 h avant passage dans l'actimètre.

Seule la dose de 512 mg/kg augmente modérément la motilité spontanée de la souris.
Le CRL 41131 augmente l'activité motrice spontanée de la souris pour des doses $\geq$ 32 mg/kg; l'intensité et la durée de l'effet croissent avec l'augmentation des doses (4 h à 32 mg/kg, 4 à 24 h à 128 mg/kg et > 24 h à 512 mg/kg). A noter le délai d'apparition de l'effet stimulant moteur pour la dose de 512 mg/kg.

10) Action sur l'agressivité intergroupes.

Après avoir séjourné pendant 3 semaines dans chacune des moitiés d'une cage séparées par une cloison opaque, des groupes de 3 souris reçoivent le CRL 41131. Trente minutes plus tard, les deux groupes d'une même cage sont réunis par retrait de la cloison et on note le nombre de combats qui surviennent en 10 min.
A la plus forte dose utilisée (512 mg/kg), le CRL 41131 diminue nettement le nombre de combats.

11) Action vis-à-vis de quelques comportements perturbés par divers agents.

1 - Motilité réduite par habituation à l'enceinte.

Après 18 h de séjour dans les actimètres, les souris (6 par dose, 12 témoins) reçoivent le CRL 41131. Elles sont aussitôt replacées dans leurs enceintes respectives et, 30 min plus tard, on enregistre leur motilité pendant 30 min.
Aux doses de 32, 128 et 512 mg/kg, le CRL 41131 entraîne une reprise nette de l'activité motrice chez la souris habituée à son enceinte.

2 - Motilité réduite par agression hypoxique.

Trente minutes après avoir reçu le CRL 41131, les souris (10 par dose, 20 témoins) sont soumises à une anoxie hypobare aiguë (dépression de 600 mmHg en 90 s, détente de 45 s), puis elles sont placées en actimètre où leur motilité est enregistrée pendant 10 min.
Aux doses de 8, 32, mais surtout 128 et 512 mg/kg, le CRL 41131 entraîne une amélioration de la récupération motrice chez la souris dont la motilité a été déprimée à la suite d'un séjour bref dans une enceinte à pression réduite.

3 - Anoxie asphyxique

Des lots de 10 souris reçoivent le CRL 41131 30 min avant l'administration intrapéritonéale de 32 mg/kg de triiodoéthylate de gallamine.
Le CRL 41131 ne modifie pratiquement pas le délai d'apparition des convulsions consécutives à une anoxie asphyxique provoquée par un curarisant.

12) Interaction avec le barbital.

Trente minutes après l'administration de CRL 41131, des lots de 10 souris reçoivent une injection intrapéritonéale de barbital (220 mg/kg).
Aux doses de 128 et 512 mg/kg, le CRL 41131 diminue la durée du sommeil barbiturique.

13) Action sur le "Désespoir comportemental".

Trente min après avoir reçu le CRL 41131, des lots de 6 souris (mâles, $CD_1$, Charles River) sont placés dans un bécher rempli d'eau sur une hauteur de 6 cm. On note la durée totale d'immobilité entre la deuxième et la sixième minute suivant l'immersion.

Aux doses de 32, 128, 512 mg/kg, le CRL 41131 diminue la durée de l'immobilité de la souris placée en immersion forcée.

14) <u>Recherche d'une toxicité particulière chez les souris groupées</u>.

Aussitôt après l'administration de CRL 41131, les souris groupées par lots de 10 sont placées dans des boîtes en Plexiglass (20 × 10 × 10 cm).

On note le nombre d'animaux morts, toutes les heures pendant 4 h et après 24, 48 et 72 h.

La toxicité du CRL 41131 est déterminée dans les mêmes conditions sur des lots de souris placées à raison d'une par boîte.

Dans les conditions habituelles d'évaluation de la toxicité (24 h après administration des substances), la dose létale 50% ($DL_{50}$) du CRL 41131 est estimée à :
- 700 mg/kg chez les souris groupées;
- 1 500 mg/kg chez la souris isolée;

$$\text{Le rapport } \frac{DL_{50} \text{ isolées}}{DL_{50} \text{ groupées}} = 2{,}14$$

Le CRL 41131 est donc modérément plus toxique chez les souris groupées que ches les souris isolées.

A titre de comparaison, le rapport

$$\frac{DL_{50} \text{ isolée}}{DL_{50} \text{ groupées}}$$

déterminé dans les mêmes conditions est voisin de 8 pour l'amphétamine, 4 pour la benzophétamine, 8 pour le diéthylpropion.

Cependant, l'état général des animaux survivants à 24 h a conduit à prolonger la période d'observation.

La $DL_{50}$ du CRL 41131, 48 h après administration, peut être estimée à :
- 480 mg/kg chez les souris groupées,
- 1 300 mg/kg chez les souris isolées,

et, 72 h après administration, à :
- 370 mg/kg chez les souris groupées,
- 1 100 mg/kg chez les souris isolées.

$$\text{Le rapport } \frac{DL_{50} \text{ isolée}}{DL_{50} \text{ groupées}}$$

est de 2,70 à 48 h, 2,97 à 72 h.

Les résultats qui précèdent font apparaître que, dans les conditions expérimentales utilisées, le CRL 41131 présente des effets de type stimulant chez la souris et le rat.

a) - <u>Chez la souris</u> - excitation
- augmentation des réactivités
- hypermotilité
- reprise de l'activité motrice chez la souris habituée à son enceinte
- amélioration de la récupération motrice chez la souris placée en hypoxie
- diminution de l'immobilité de "désespoir"
- existence d'une toxicité particulière chez les souris groupées.

b) - <u>Chez le rat</u> - excitation
- présence de mouvements stéréotypés
- potentialisation des stéréotypies induites par l'apomorphine et l'amphétamine.

Cet effet stimulant semble se développer à doses élevées, après une période de latence importante, de l'ordre de 2 h au moins. Ce délai nécessaire au plein développement de l'effet du CRL 41131 est peut-être responsable des résultats négatifs obtenus au niveau de certains tests : l'antagonisme tardif (24 h) de l'hypothermie et du ptôsis réserpiniques peut laisser supposer que le CRL 41131 possède une activité antidépressive potentielle et exerce des effets stimulants $\alpha$-adrénergiques.

Conformément à l'invention, on a préparé par les procédés habituels des compositions thérapeutiques renfermant, en association avec un excipient physiologiquement acceptable, l'acide N,N-bis-(4-fluorophényl)carbamoylacétohydroxamique.

Le CRL 41131 a donné de bons résultats comme antidépresseur stimulant chez l'homme à la dose de 2 à 3 comprimés par jour, à 150 mg, par voie buccale.

Comparaison des propriétés pharmacologiques du CRL 41131 et du CRL 40385

Dans les conditions habituelles d'évaluation de la toxicité (24 heures après administration des substances), la dose léthale 50 % ($DL_{50}$ du CRL 40385 est estimée à :
- 1050 mg/kg chez les souris groupées ;
- 1500 mg/kg chez la souris isolée ;

$$\text{Le rapport } \frac{DL_{50} \text{ isolées}}{DL_{50} \text{ groupées}} = 1,43$$

Comme mentionné précédemment, dans les mêmes conditions, le CRL 41131 présente un rapport :

$$\frac{DL_{50} \text{ isolées}}{DL_{50} \text{ groupées}} = 2,14$$

ce qui constitue une différence importante dans des mêmes conditions expérimentales.

Sur le test à l'amphétamine, le CRL 40385 prolonge les stéréotypies du rat, tandis que le CRL 41131 potentialise ces stéréotypies.

Sur le test à la réserpine, le CRL 40385 s'oppose modérément à 64 mg/kg, tandis que le CRL 41131 s'oppose tardivement à cette hypothermie à 128 et 512 mg/kg.

Sur le test à l'oxotrémorine, le CRL 40385 antagonise l'hyperthermie et diminue l'intensité des tremblements, tandis que le CRL 41131 n'a pas d'effet.

Sur le test à l'électrochoc, le CRL 40385 s'oppose aux convulsions, tandis que le CRL 41131 au contraire aggrave les effets létaux de l'électrochoc.

En ce qui concerne l'agressivité intergroupe des souris, on constate une augmentation avec le CRL 40385 (32 et 256 mg/kg) et une diminution avec le CRL 41131 (128 et 512 mg/kg).

Avec l'association au barbital, le CRL 40385 diminue d'abord le sommeil, mais à la dose de 512 mg/kg, il ne le fait plus. Contrairement, le CRL 41131 diminue encore le sommeil à 128 et 512 mg/kg.

Cette comparaison met en évidence les différences tout à fait inattendues, existant entre l'acide N,N-bis-(4-fluorophényl) carbamoylacétohydroxamique selon l'invention, et le CRL 40385, composé le plus proche de l'état de la technique.

**Revendications pour les Etats contractants BE, CH, DE, FR, GB, IT, LI, LU, NL, SE**

1. Acide acétohydroxamique, caractérisé en ce qu'il s'agit de l'acide N,N-bis-(4-fluorophényl)carbamoylacétohydroxamique de formule :

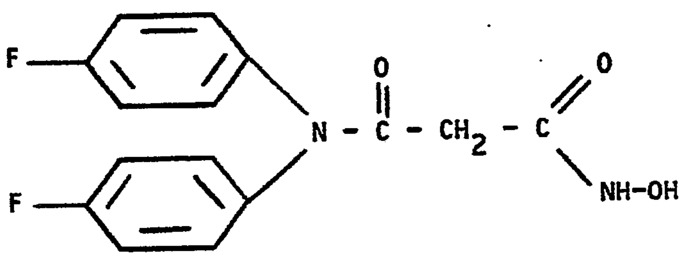

2. Composition thérapeutique, caractérisée en ce qu'elle renferme, en association avec un excipient pharmacologiquement acceptable, l'acide N,N-bis-(4-fluorophényl)carbamoylacétohydroxamique, en tant qu'agent actif notamment antidépresseur stimulant.

**Revendications pour l'Etat contractant AT**

1. Acide acétohydroxamique, caractérisé en ce qu'il s'agit de l'acide N,N-bis-(4-fluorophényl)carbamoylacétohydroxamique de formule :

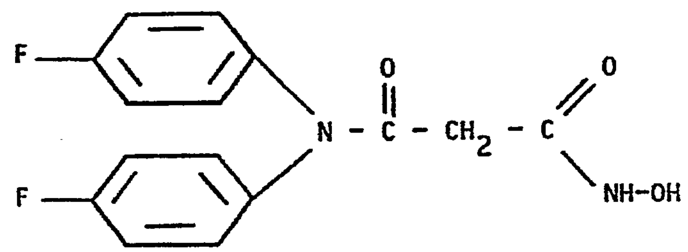

2. Composition thérapeutique, caractérisée en ce qu'elle renferme, en association avec un excipient pharmacologiquement acceptable, l'acide N,N-bis-(4-fluorophényl)carbamoylacétohydroxamique, en tant qu'agent actif notamment antidépresseur stimulant.

3. Procédé d'obtention de l'acide N,N-bis-(4-fluorophényl) carbamoylacétohydroxamique de formule :

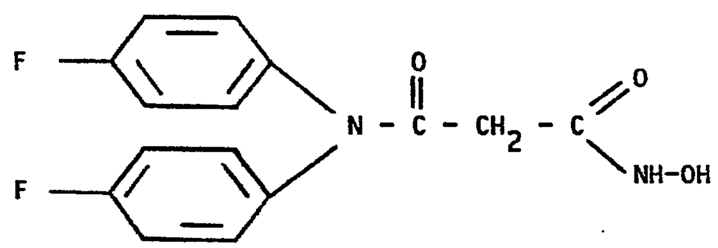

caractérisé en ce qu'il consiste à faire réagir du chlorure de méthylmalonyle et de la 4,4'-difluorodiphénylamine pour obtenir du N,N-bis-(4-fluorophényl)carbamoylacétate de méthyle, à préparer une solution méthanolique d'hydroxylamine dans laquelle on ajoute le N,N-bis-(4-fluorophényl)carbamoylacétate de méthyle et à récupérer après chauffage, évaporation, extraction et recristallisation ledit acide N,N-bis-(4-fluorophényl) carbamoylacétohydroxamique.

4. Utilisation de l'acide N,N-bis-(4-fluorophényl) carbamoylacétohydroxamique pour la réalisation d'un médicament notamment actif comme antidépresseur stimulant.

Revendications pour l'Etat contractant GR

1. Acide acétohydroxamique, caractérisé en ce qu'il s'agit de l'acide N,N-bis-(4-fluorophényl)carbamoylacétohydroxamique de formule :

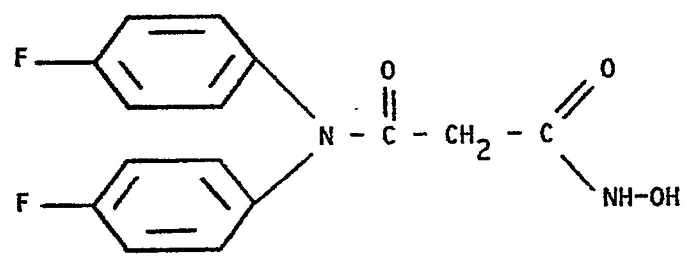

2. Procédé d'obtention de l'acide N,N-bis-(4-fluorophényl) carbamoylacétohydroxamique de formule :

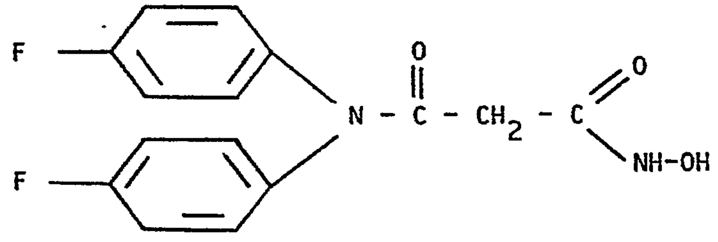

caractérisé en ce qu'il consiste à faire réagir du chlorure de méthylmalonyle et de la 4,4'-difluorodiphénylamine pour obtenir du N,N-bis-(4-fluorophényl)carbamoylacétate de méthyle, à préparer une solution méthanolique d'hydroxylamine dans laquelle on ajoute le N,N-bis-(4-fluorophényl)carbamoylacétate de méthyle et à récupérer après chauffage, évaporation, extraction et recristallisation ledit acide N,N-bis-(4-fluorophényl) carbamoylacétohydroxamique

3. Utilisation de l'acide N,N-bis-(4-fluorophényl) carbamoylacétohydroxamique pour la réalisation d'un médicament notamment actif comme antidépresseur stimulant.

Revendications pour l'Etat contractant ES

1. Procédé d'obtention de l'acide N,N-bis-(4-fluorophényl) carbamoylacétohydroxamique de formule :

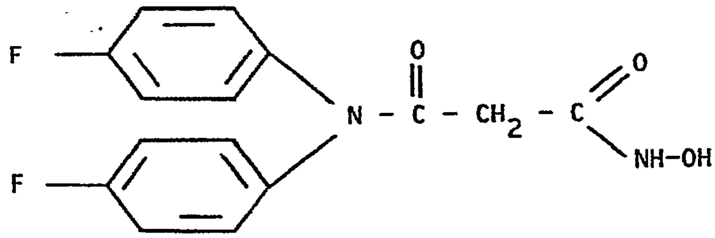

caractérisé en ce qu'il consiste à faire réagir du chlorure de méthylmalonyle et de la 4,4'-difluorodiphénylamine pour obtenir du N,N-bis-(4-fluorophényl)carbamoylacétate de méthyle, à préparer une solution méthanolique d'hydroxylamine dans laquelle on ajoute le N,N-bis-(4-fluorophényl)carbamoylacétate de méthyle et à récupérer après chauffage, évaporation, extraction et recristallisation ledit acide N,N-bis-(4-fluorophényl) carbamoylacétohydroxamique.

2. Utilisation de l'acide N,N-bis-(4-fluorophényl) carbamoylacétohydroxamique pour la réalisation d'un médicament notamment actif comme antidépresseur stimulant.

**Patentansprüche für die Vertragsstaaten BE, CH, DE, FR, GB, IT, LI, LU, NL, SE:**

1. Acetohydroxamsäure, dadurch gekennzeichnet, daß es sich um die N,N-bis-(4-Flourphenyl)-carbamoylacetohydroxamsäure der Formel

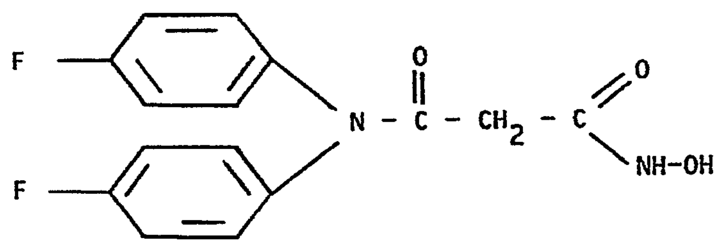

handelt.

2. Therapeutische Zusammensetzung, dadurch gekennzeichnet, daß sie in Kombination mit einem pharmakologisch akzeptablen Exzipienten N,N-bis-(4-Flourphenyl)-carbamoylacetohydroxamsäure als Wirkstoff, insbesondere als stimulierendes Antidepressivum, enthält.

**Patentansprüche für den Vertragsstaat AT:**

1. Acetohydroxamsäure, dadurch gekennzeichnet, daß es sich um die N,N-bis-(4-Fluorphenyl)-carbamoylacetohydroxamsäure der Formel

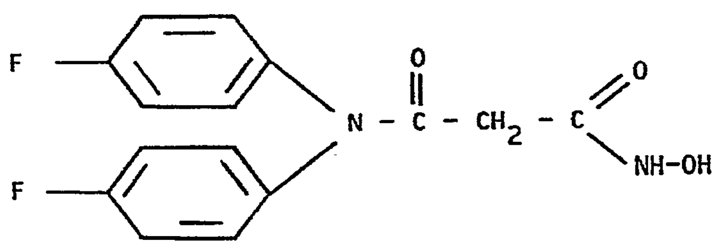

handelt.

2. Therapeutische Zusammensetzung, dadurch gekennzeichnet, daß sie in Kombination mit einem pharmakologisch akzeptablen Exzipienten N,N-bis-(4-Fluorphenyl)-carbamoylacetohdroxamsäure als Wirkstoff, insbesondere als stimulierendes Antidepressivum, enthält.

3. Verfahren zur Herstellung von N,N-bis-(4-Fluorphenyl)-carbamoylacetohydroxamsäure der Formel

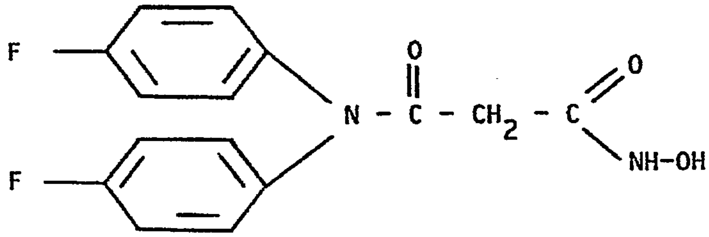

dadurch gekennzeichnet, daß es darin besteht, daß Methylmalonylchlorid und 4,4'-Difluordiphenylamin zur Herstellung von Methyl-N,N-bis-(4-Fluorphenyl)-carbamoylacetat zur Umsetzung gebracht werden, eine methanolische Hydroxylaminlösung bereitet wird, in die man Methyl-N,N-bis-(4-Fluorphenyl)-carbamoylacetat gibt, und nach Erhitzen, Eindampfen, Extrahieren und Umkristallisieren die N,N-bis-(4-Fluorphenyl)-carbamoylacetohydroxamsäure gewonnen wird.

4. Verwendung von N,N-bis-(4-Fluorphenyl)-carbamoylacetohydroxamsäure zur Herstellung eines Medikaments, das insbesondere als stimulierendes Antidepressivum wirkt.

**Patentansprüche für den Vertragsstaat ES:**

1. Verfahren zur Herstellung von N,N-bis-(4-Fluorphenyl)-carbamoylacetohydroxamsäure der Formel

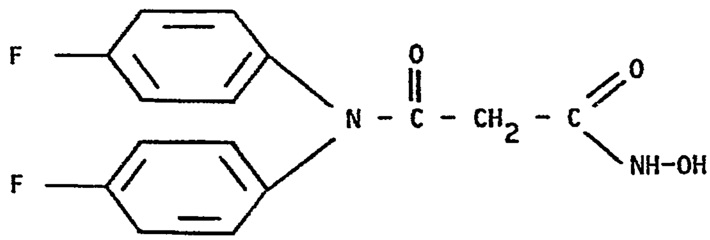

dadurch gekennzeichnet, daß es darin besteht, daß Methylmalonylchlorid und 4,4'-Difluordiphenylamin zur Herstellung von Methyl-N,N-bis-(4-Fluorphenyl)-carbamoylacetat zur Umsetzung gebracht werden, eine methanolische Hydroxylaminlösung bereitet wird, in die man Methyl-N,N-bis-(4-Fluorphenyl)-carbamoylacetat gibt, und nach Erhitzen, Eindampfen, Extrahieren und Umkristallisieren die N,N-bis-(4-Fluorphenyl)-carbamoylacetohydroxamsäure gewonnen wird.

2. Verwendung von N,N-bis-(4-Fluorphenyl)-carbamoylacetohydroxamsäure zur Herstellung eines Medikaments, das insbesondere als stimulierendes Antidepressivum wirkt.

**Patentansprüche für den Vertragsstaat GR:**

1. Acetohydroxamsäure, dadurch gekennzeichnet, daß es sich um die N,N-bis-(4-Fluorphenyl)-carbamoylacetohydroxamsäure der Formel

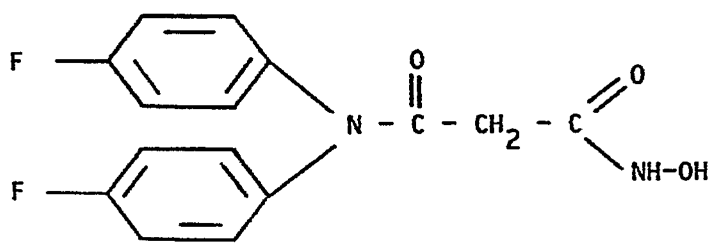

handelt.

2. Verfahren zur Herstellung von N,N-bis-(4-Fluorphenyl)-carbamoylacetohydroxamsäure der Formel

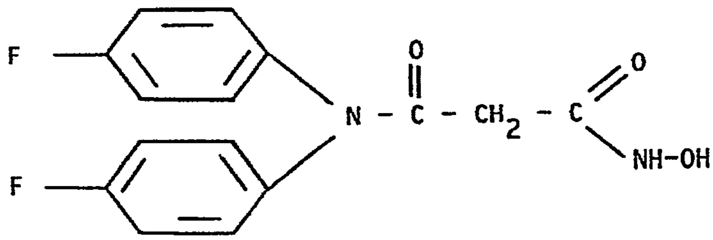

dadurch gekennzeichnet, daß es darin besteht, daß Methylmalonylchlorid und 4,4'-Difluordiphenylamin zur Herstellung von Methyl-N,N-bis-(4-Fluorphenyl)-carbamoylacetat zur Umsetzung gebracht werden, eine methanolische Hydroxylaminlösung bereitet wird, in die man Methyl-N,N-bis-(4-Fluorphenyl)-carbamoylacetat gibt, und nach Erhitzen, Eindampfen, Extrahieren und Umkristallisieren die N,N-bis-(4-Fluorphenyl)-carbamoylacetohydroxamsäure gewonnen wird.

3. Verwendung von N,N-bis-(4-Fluorphenyl)-carbamoylacetohydroxamsäure zur Herstellung eines Medikaments, das insbesondere als stimulierendes Antidepressivum wirkt.l

**Claims for contracting states: BE, CH, DE, FR, GB, IT, LI, LU, NL, SE**

1. Acetohyroxamic acid, characterized in that it is the N,N-bis-(4-fluorophenyl)carbamoylacetohyroxamic acid of formula:

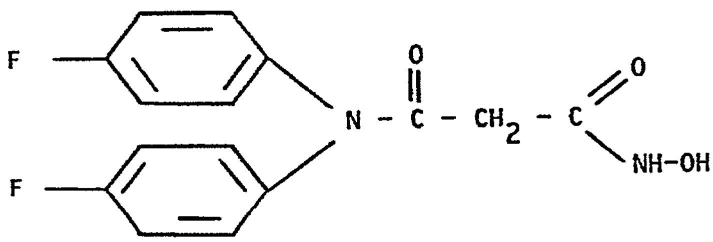

2. Therapeutic composition, characterized in that it contains in combination with a pharmacologically acceptable excipient, the N,N-bis-(4-fluorophenyl)carbamoylacetohydroxamic acid as active agent, particularly as stimulant antidepressant.

**Claims for the contracting state: AT**

1. Acetohydroxamic acid, characterized in that it is the N,N-bis-(4-fluorophenyl)carbamoylacetohydroxamic acid, of formula:

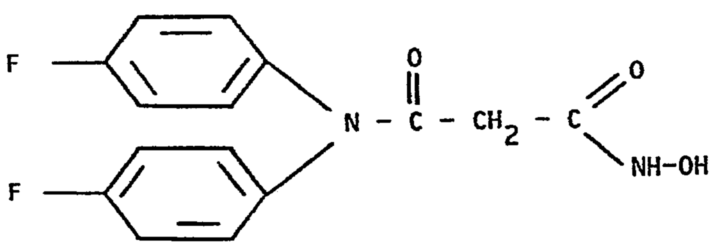

2. Therapeutic composition, characterized in that it contains in combination with a pharmacologically acceptable excipient, the N,N-bis-(4-fluorophenyl)carbamoylacetohydroxamic acid as active agent, particularly as stimulant antidepressant.

3. Process for preparing N,N-bis-(4-fluorophenyl)carbamoylacetohydroxamic acid of formula:

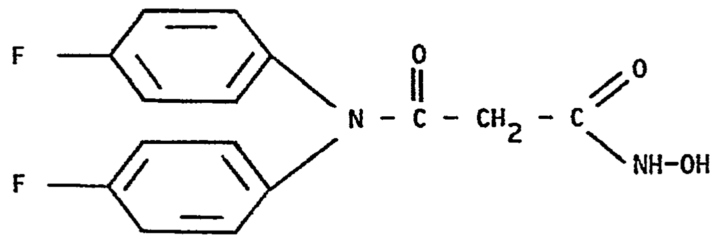

characterized in that it consists in reacting methylmalonyl chloride with 4,4'-difluorodiphenylamine in order to obtain methyl N,N-bis-(4-fluorophenyl)carbamolacetate in preparing a methanolic solution of hydroxylamine in which the methyl N,N-bis-(4-fluorophenyl)carbamoylacetate is added and in recovering said N,N-bis-(4-fluorophenyl)carbamoylacetohydroxamic acid after treating, evaporation, extraction and recrystallization.

4. Use of N,N-bis-(4-fluorophenyl)carbamoylacetohydroxamic acid for preparing a drug which is particulary active as stimulant antidepressant.

**Claims for contracting states: ES**

1. Process for preparing N,N-bis-(4-fluorophenyl)carbamoylacetohydroxamic acid of formula:

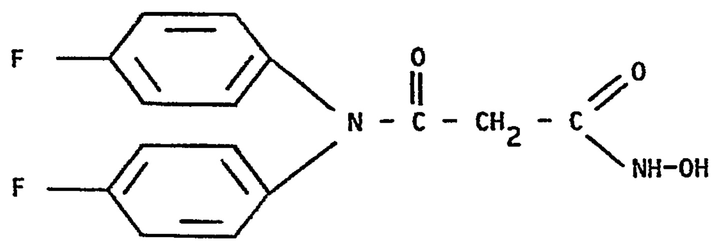

characterized in that it consists in reacting methylmalonyl chloride with 4,4'-difluorodiphenylamine, in order to obtain methyl N,N-bis-(4-fluorophenyl)carbamoylacetate in preparing a methanolic solution of hydroxylamine in which the methyl N,N-bis-(4-fluorophenyl)carbamoylacetate is added and in recovering said N,N-bis-(4-fluorophenyl)carbamoylacetohydroxamic acid after heating, evaporation, extraction and recrystallization.

2. Use of N,N-bis(4-fluorophenyl)carbamoylacetohydroxamic acid for preparing a drug which is particularly active as a stimulant antidepressant.

**Claims for contracting state: GR**

1. Acetohydroxamic acid, characterized in that it is the N,N-bis-(4-fluorophenyl)-carbamoylacetohydroxamic acid of formula:

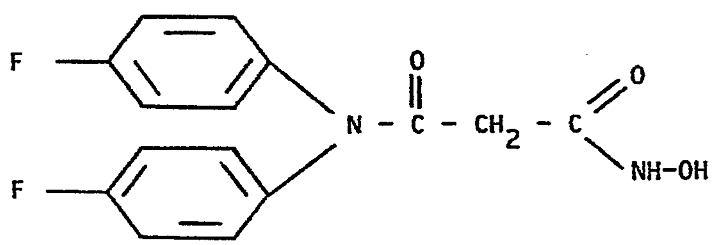

2. Process for preparing N,N-bis-(4-fluorophenyl)carbamoylacetohydroxamic acid of formula:

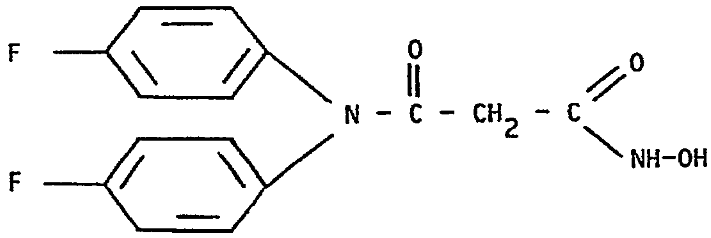

characterized in that it consists in reacting methylmalonyl chloride with 4,4'-difluorodiphenylamine in order to obtain methyl-N,N-bis-(4-fluorophenyl)carbamoylacetate in preparing a methanolic solution of hydroxylamine in which the methyl N,N-bis-(4-fluorophenyl)carbamoylacetate is added and said N,N-bis-(4-fluorophenyl)-carbamoylacetohydroxamic acid after heating, evaporation, extraction and recrystallization.

3. Use of N,N-bis-(4-fluoropheny)carbamoylacetohydroxamic acid for preparing a drug which is particularly active as a stimulant antidepressant.